# EUROPEAN PATENT SPECIFICATION

(11) **EP 4 482 441 B1**
(45) Date of publication and mention of the grant of the patent: **09.12.2026**
(21) Application number: 23708869.5
(22) Date of filing: 22.02.2023
(51) Int. Cl.: A61F 5/44

(54) **AN OSTOMY DRAINAGE SYSTEM**
STOMA-DRAINAGESYSTEM
SYSTÈME DE DRAINAGE DE STOMIE

(30) Priority: 22.02.2022 GB 202202398
(43) Date of publication of application: 01.01.2025
(73) Proprietor: Salts Healthcare Limited, Birmingham, West Midlands B7 4AA (GB)
(72) Inventor: HOWARD, Lee, Birmingham, West Midlands B7 4AA (GB); WILLIAMS, Kieran, Birmingham, West Midlands B7 4AA (GB); SMITH, Lee, Birmingham, West Midlands B7 4AA (GB)
(74) Representative: Forresters IP LLP
(86) International application number: PCT/GB2023/050392
(87) International publication number: WO 2023/161618

(56) References cited:
- WO-A1-2020/174220
- US-A1- 2012 130 329
- US-A1- 2017 156 919

## Description

Urostomy appliances are well known in the field. They are typically attached to a user via an adhesive member that extends around the user's stoma with adhesive and provide a collecting volume to collect waste (mostly liquid waste) exiting the stoma. A mechanism for draining the collecting volume is often provided - typically, these are in the form of a tap, so that a user can empty waste that is collected in the collecting volume without having to remove and dispose of the entire appliance. US2012/130329 discloses a valve that is sealable to an associated container having an interior fluid storage region is adapted to permit and stop flow of fluid from the fluid storage region.

At times, a user may want to extend the time between emptying their appliance, for example, when they are sleeping. Typically, a larger capacity bag is used to contain waste while the user is sleeping. In this scenario, the outlet valve is connected to the larger capacity bag by a tube, so that the bag fills with waste.

Embodiments of the present invention seek to alleviate one or more problems associated with the prior art.

### BRIEF DESCRIPTION OF THE INVENTION

According to a first aspect of the invention we provide an ostomy drainage system including an ostomy appliance and a drainage conduit, the ostomy appliance including:
an internal collecting volume defined by a first wall and a second wall connected together about a periphery;
a stoma receiving opening defined in the first wall, and
a valve including:
   a body connected to the first and second walls,
   an inlet and an outlet, connected by a flow path and fluidly connected to the internal collecting volume; and
the drainage conduit including a connector device which is connectable to the valve, wherein the connector device has a connector body providing a passage, and an inlet formation which widens radially outwardly relative to the connector body, such that when the connector device is connected to the valve, the connector device is inserted into the outlet of the valve, and the connector body extends from the outlet to the inlet and extends through the inlet into the interior of the ostomy appliance, and the inlet formation resists removal from the valve.

The inlet formation may form part of a mucus guard member. The connector device may be connected to the valve. The connector body may extend from the outlet to the inlet. The mucus guard member may be located at the inlet and may provide a cage around the inlet to inhibit the ingress of mucus. The mucus guard member may include a generally upstanding wall which, in use, projects away from the inlet of the valve. The wall may project from the connector body in an annular shape. The mucus guard member may include a plurality of slots or channels, and optionally the slots / channels may be spaced around the inlet. The mucus guard member may have four slots. The first and second slots may be separated by a bar of the cage and form a first pair and the third and fourth slots may be separated by a second bar of the cage and form a second pair. The first pair may be on an opposing side of the mucus guard member to the second pair. The mucus guard member may form a frustoconical shape.

The shape of the mucus guard member may be wider at a base and tapers to an end. The base may be between 0.8 and 1.2 cm wide and preferably around 0.95 cm wide. The end of the mucus guard member may be between 0.7 and 0.95 cm wide.

The connector body may include a shoulder formation. The shoulder formation may be positioned adjacent the outlet of the valve when in use. The connector body and / or the mucus guard member may be formed from a rigid material and optionally a rigid plastics material. The connector body and the mucus guard member may be formed from the same material.

The mucus guard member may be between 0.8 and 1.2 cm long and preferably around 1cm long. The connector body may be between 0.9 and 1.3 cm long and preferably around 1.1cm long. A ratio of the length of the connector body to the length of the mucus guard member may be between 1:1 and 1.5:1 and may be preferably around 1.1:1.

The connector device may provide an anchor that holds the connector device connected to the valve and a mucus guard member that prevents / reduces how much mucus travels into the passage.

### BRIEF DESCRIPTION OF THE FIGURES

In order that the present disclosure may be more readily understood, preferable embodiments thereof will now be described, by way of example only, with reference to the accompanying drawings, in which:
FIGURE 1 is a schematic view of an ostomy drainage system;
FIGURE 2 is a schematic side cross-sectional view of an ostomy appliance;

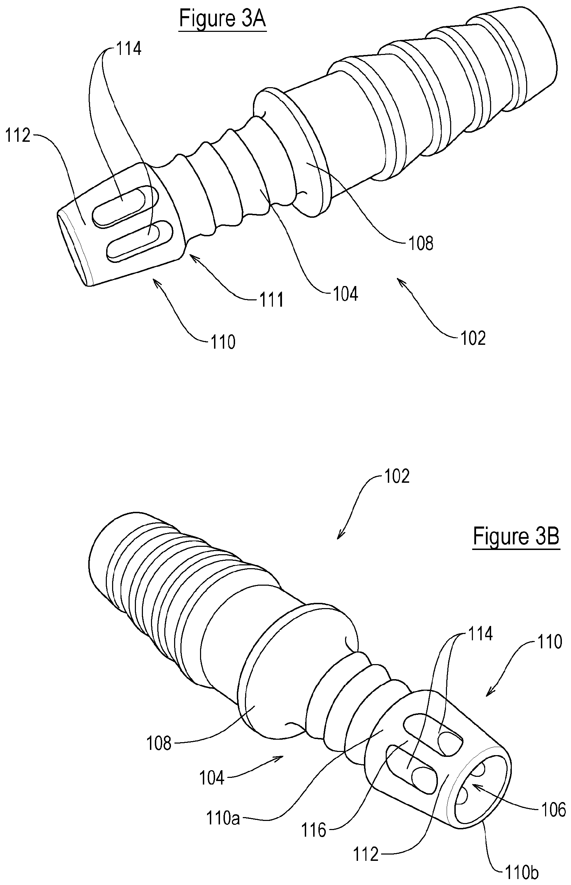

FIGURES 3A and 3B show different views of part of a drainage conduit;
FIGURE 4 is a perspective view of part of the drainage conduit and a valve of an ostomy appliance; and
FIGURE 5 shows part of the drainage conduit connected to the valve of the ostomy appliance.

### DETAILED DESCRIPTION OF THE DISCLOSURE

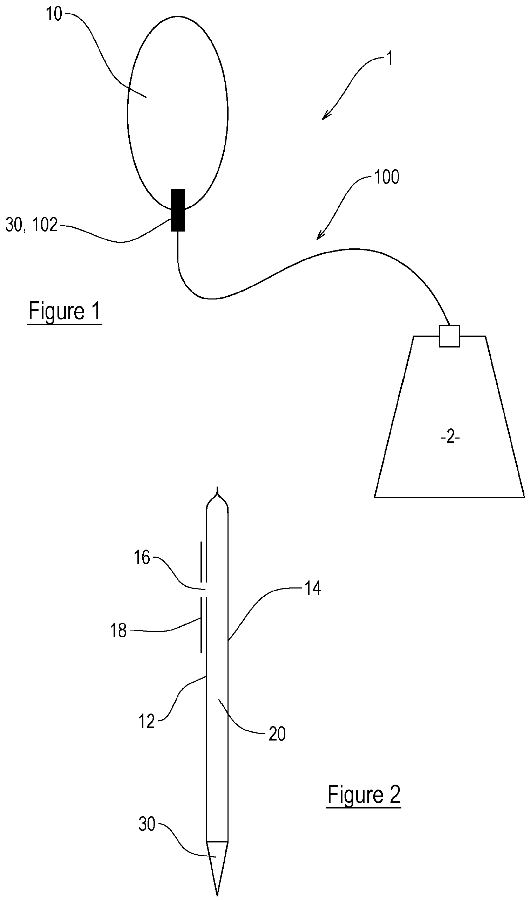

Referring to the figure 1, an ostomy drainage system 1 is illustrated. The ostomy drainage system 1 includes an ostomy appliance 10 and a drainage conduit 100.

Referring to figure 2, an ostomy appliance 10 (or appliance 10) is illustrated. The ostomy appliance 10 includes a first wall 12 and a second wall 14. The first and second wall 12, 14 are connected together about a periphery to define an internal collecting volume 20. In the illustrated example, the first wall 12 is on a "body" side of the appliance 10 (i.e. it is closer to the body than the internal collecting volume when the appliance 10 is in use) and the second wall 14 is on a "non-body" side of the appliance 10 (i.e. it is further / furthest from the body than the internal collecting volume when the appliance 10 is in use).

A stoma receiving opening 16 is defined in the first wall 12. The stoma receiving opening 16 is in fluid communication with the internal collecting volume such that in use, waste entering the appliance via the stoma receiving opening flows into the collecting volume. In some embodiments, an adhesive member 18 is attached to the first wall 12 and extends around the stoma receiving opening 16. The adhesive member 18 permits a user of the appliance 10 to attach the appliance 10 to their abdomen around their stoma.

A valve 30 is provided to allow waste to be emptied from the internal collecting volume 20. The valve 30 includes a body 32, an inlet 40 and an outlet 42. The body 32 is connected to the first and second walls 12, 14. The inlet 40 and the outlet 42 are connected by a flow path and are fluidly connected to the internal collecting volume 20.

The valve 30 includes a closure member (not shown) which is used to close the valve 30. The closure member is inserted into the outlet 42 and blocks the flow path out of the appliance 10. When a user wishes to open the valve 30 (i.e. to empty their appliance 10 or to connect a drainage conduit 100 as discussed below), the closure member is removed from the outlet 42 so that the flow path is open and waste can flow from the internal collecting volume 20 through the valve and out of the appliance 10.

As mentioned above, the drainage system 1 further includes a drainage conduit 100. The drainage conduit 100 includes a connector device 102 which is connectable to the valve 10. The connector device 102 has a connector body 104 and an inlet formation 111. In some embodiments, the inlet formation 111 forms part of a mucus guard member 110.

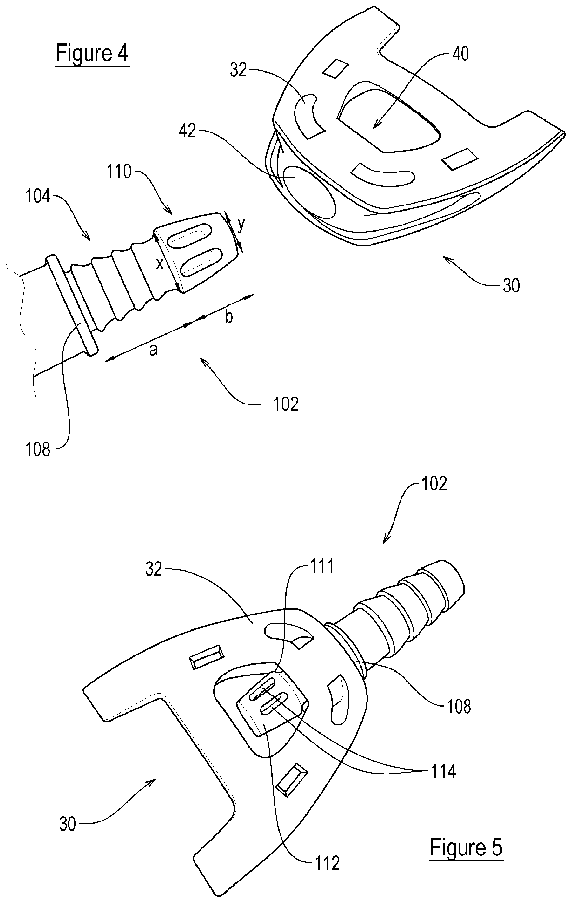

The connector body 104 has a central bore that provides a passage 106. An exterior surface of the connector body 104 includes multiple tapered surfaces, each of which extends axially along the connector body 104 and is adjacent the next tapered surface. Thus, the exterior surface of the connector body 104 has a series of wedge shaped parts that form the surface. In some embodiments, the connector body 104 is between 0.9 and 1.3 cm long and preferably around 1.1cm long (illustrated by "a" in figure 4).

In some embodiments, the connector body 104 includes a shoulder formation 108. In other words, the connector body 104 has an outwardly projecting flange that extends around the body 104. The shoulder formation 108 is spaced from the mucus guard member 110 (and, in the illustrated example, the tapered exterior surface of the connector body 104 is located adjacent the shoulder formation 108 and between the shoulder formation 108 and the mucus guard member 110).

The inlet formation 111 widens radially outwardly relative to the connector body 104. In other words, the inlet formation 111 provides a conical or outwardly tapering part that extends outwardly of the connector body 104. The inlet formation 111 may form part of the mucus guard member 110 described below or it may be provided alone.

The mucus guard member 110 provides a cage around an end of the connector body 104 (i.e. a formation that provides multiple "bars" or projections away from the connector body 104 that catches / slows mucus flowing towards the passage 106). The mucus guard member 110 provides a flow path into the connector body 104 / conduit 100 for liquid waste but inhibits the progress of more viscous material into the passage 106. In other words, the end of the drainage conduit 100 has a sieve-like / filter formation around it to prevent (or at least inhibit and slow down) the ingress of mucus into the conduit 100.

In some embodiments, the mucus guard member 110 forms a frustoconical shape. In other words, a base 110a of the mucus guard member 110 is wider than an end 110b. The mucus guard member 110 tapers gradually from the base 110a to the end 110b. In the present example, the base 11a is between 0.8 and 1.2 cm wide and preferably around 0.95 cm wide (see line "x" on figure 4). Further, the end 110b of the mucus guard member 110 is between 0.5 and 0.9 cm wide and preferably around 0.7 cm wide (see line "y" on figure 4). It should be appreciated that the mucus guard member 110 may not form a frustoconical shape - in which case both the base and the end may be around 0.95cm.

In some embodiments, the mucus guard member 110 is between 0.8 and 1.2 cm long and preferably around 1cm long (see line "b" in figure 4). Thus, a ratio of the length of the connector body (line "a") to the length of the mucus guard member (line "b") is between 1:1 and 1.5:1. In the present example, the total length of the connector device 102 is around 2.1 cm, where the connector body 104 is around 1.1cm and the mucus guard member 110 is around 1.0cm (thus, the ratio of body 104 length to member 110 length is around 1.1:1).

It should be appreciated that the mucus guard member 110 could have a longer length and provide more apertures to form the sieve / filter for the mucus. For example, it could be up to 30 to 50 mm. In such an example, the material may be softer (e.g. a TPE) and the length would be chosen to avoid interfering with a non-return valve within the ostomy appliance 10 itself (the non-return valve is formed of layers of film that ensures waste entering the appliance 10 moves away from the stoma area and cannot return).

In some embodiments, the mucus guard member 110 includes a generally upstanding wall 112. The upstanding wall 112 projects from the connector body 104 in an annular shape. In the present example, a circular opening is provided in the end of the mucus guard member 110. However, this need not be the case and one or more additional structural members may be provided across the passage to provide further prevention of mucus into the passage 106.

The mucus guard member 110 includes a plurality of slots 114 (and, preferably, has four slots 114) formed in the wall 112. In this context a slot is considered to be substantially elongated oval shape or a stadium shape and extends through the material of the mucus guard member 110 (i.e. through the wall 112) to provide a small opening. In some embodiments, each opening is around 6mm long and 1.9mm wide.

It should be appreciated, that each slot could be formed as a recess instead. In other words, a channel would be formed extending from the end 110b of the mucus guard member 110 towards the base 110a.

In the illustrated example, first and second slots 114 are formed adjacent each other with a structure member 116 provided in between. Further, the third and fourth slots 114 are also formed adjacent each other with a structural member 116 provided between them. In other words, the first and second slots 114 form a first pair and the third and fourth slots 114 form a second pair.

In the present example, the first pair is positioned on a first side of the mucus guard member 110 and the second pair is positioned on a second, opposing, side of the mucus guard member 110.In other words, the cage of the mucus guard member 110 mentioned above is formed from the wall 112 and the slots 114 distributed around the wall 112 (they may form an evenly distributed annular arrangement). The structural members 116 form the "bars" of the cage.

In some embodiments, each of the slots 114 is positioned the same distance from a plane that extends transversely / perpendicularly through the connector body 104. As such they lie the same distance from the inlet 40 of the valve 30 during use.

In some embodiments, the connector body 104 and / or the mucus guard member 110 are formed from a rigid material. The rigid material may preferably be a rigid plastics material.

The features of the drainage conduit 100 will now be discussed with reference to where they are located when in use (i.e. when the connector device 102 is connected to the valve 30). This is shown in figure 5.

The connector device 102 is inserted into the outlet 42 of the valve 30 and extends through the inlet 40 into the interior of the appliance 10. The connector body 104 extends from the outlet 42 to the inlet 40. Thus, the multiple tapered surfaces discussed above engage with an interior surface of the valve 30 (in the flow path between the inlet 40 and the outlet 42). The shaped exterior surface of the connecter body 104 aids the sealing contact against the valve 30 and stops leakage of waste between the valve 30 and the connector device 102.

The inlet formation 111 flares outwardly from the inlet of the valve 30 (this inlet formation 111 is wider than the inlet 40). Thus, the inlet formation 111 provides an anchor function / resists removal from the valve 30. In other words, when outward force is applied the connector device 102 (i.e. away from the outlet 42 and the appliance 10), the inlet formation 111 inhibits removal of the connector device 102 from the valve 30.

As can be seen in figure 5, the shoulder formation 108 is positioned adjacent the outlet 42 of the valve 30. This flanged portion prevents the connector body 104 from being inserted into the valve 30 too far and avoids damage or distortion of the valve body 32.

The mucus guard member 110 (which is at the end of the connector body 104) is located at or adjacent the inlet 40 and provides the case around the inlet 40 to inhibit the ingress of mucus into the passage 106.

In other words, the mucus guard member 110 is positioned over the inlet 40 so that liquid is not prevented from exiting through the inlet 40 but heavier / more viscous material is impeded by the slots 114 in the side of the mucus guard member 110.

The wall 116 projects away from the inlet 40 (and into the appliance 10). As discussed above, the mucus guard member 110 is only around 1cm long, so it does not project far into the appliance 10. It is positioned only to impede the flow of mucus out of the appliance 10.

The ostomy drainage system 1 is designed for urostomy or ileostomy use where the waste excreted by a user's stoma is liquid or predominantly liquid (i.e. urine or a urine / mucus mix). The drainage system 1 is primarily for use during the night / when a user does not want to or cannot empty their urostomy appliance. The drainage conduit 100 includes a conduit that extends to a further collecting volume 2 (i.e. in addition to the internal collecting volume 20 itself).

The present invention seeks to prevent mucus build up in the conduit (which can lead to blockages and prevents urine from reaching the further collecting volume 2). This is addressed by providing a mucus guard member 110 that forms a cage around the inlet 40 of the valve 30 (and around the passage 106 of the connector device 102). It is important that the slots 114 and passage 106 are close to the inlet 40, so that waste does not collect near the valve 30 and potentially cause leaking but also so that the mucus is held out of the passage 106 and the liquid can flow more freely.

When used in this specification and claims, the terms "comprises" and "comprising" and variations thereof mean that the specified features, steps or integers are included. The terms are not to be interpreted to exclude the presence of other features, steps or components.

The invention may also broadly consist in the parts, elements, steps, examples and/or features referred to or indicated in the specification individually or collectively in any and all combinations of two or more said parts, elements, steps, examples and/or features. In particular, one or more features in any of the embodiments described herein may be combined with one or more features from any other embodiment(s) described herein.

Protection may be sought for any features disclosed in any one or more published documents referenced herein in combination with the present disclosure.

Although certain example embodiments of the invention have been described, the scope of the appended claims is not intended to be limited solely to these embodiments. The claims are to be construed literally, purposively, and/or to encompass equivalents.

## Claims

1. An ostomy drainage system (1) including an ostomy appliance (10) and a drainage conduit (100), the ostomy appliance (10) including:
an internal collecting volume (20) defined by a first wall (12) and a second wall (14) connected together about a periphery;
a stoma receiving opening (16) defined in the first wall (12), and
a valve (30) including:
a body (32) connected to the first and second walls (12, 14),
an inlet (40) and an outlet (42), connected by a flow path and fluidly connected to the internal collecting volume (20); and
the drainage conduit (100) including a connector device (102) which is connectable to the valve (30), wherein the connector device (102) has a connector body (104) providing a passage (106), and an inlet formation (111) which widens radially outwardly relative to the connector body (104), such that when the connector device (102) is connected to the valve (30), the connector device (102) is inserted into the outlet (42) of the valve (30), and the connector body (104) extends from the outlet (42) to the inlet (40) and extends through the inlet (40) into the interior of the ostomy appliance (10), and the inlet formation (111) resists removal from the valve (30).

2. An ostomy drainage system (1) according to claim 1 wherein the inlet formation (111) forms part of a mucus guard member (110), and optionally, wherein, when the connector device (102) is connected to the valve (30), the connector body (104) extends from the outlet (42) to the inlet (40) and the mucus guard member (110) is located at the inlet (40) and provides a cage around the inlet (40) to inhibit the ingress of mucus.

3. An ostomy drainage system (1) according to claim 2 wherein the mucus guard member (110) includes a generally upstanding wall which, in use, projects away from the inlet (40) of the valve (30), and optionally wherein the wall projects from the connector body (104) in an annular shape.

4. An ostomy drainage system (1) according to any one of claims 2 to 3 wherein the mucus guard member (110) includes a plurality of slots or channels, and optionally the slots / channels are spaced around the inlet (40).

5. An ostomy drainage system (1) according to claim 4 where in the mucus guard member (110) has four slots, and optionally wherein the first and second slots are separated by a bar of the cage and form a first pair and the third and fourth slots are separated by a second bar of the cage and form a second pair.

6. An ostomy drainage system (1) according to claim 5 wherein the first pair is on an opposing side of the mucus guard member (110) to the second pair.

7. An ostomy drainage system (1) according to any one of claims 2 to 6 wherein the mucus guard member (110) forms a frustoconical shape which is wider at a base and tapers to an end, and optionally, wherein the base is between 0.8 and 1.2 cm wide and preferably around 0.95 cm wide.

8. An ostomy drainage system (1) according to claim 7 wherein the end of the mucus guard member (110) is between 0.7 and 0.95 cm wide.

9. An ostomy drainage system (1) according to any one of the preceding claims wherein the connector body (104) includes a shoulder formation (111) that is positioned adjacent the outlet (42) of the valve (30) when in use, and / or wherein the connector body (104) is formed from a rigid material and optionally a rigid plastics material

10. An ostomy drainage system (1) according to any one of claims 2 to 8 or claim 9 when further dependent on any one of claims 2 to 8 wherein the mucus guard member (110) is formed from a rigid material and optionally a rigid plastics material.

11. An ostomy drainage system according to claim 10 wherein the connector body (104) and the mucus guard member (110) is formed from the same material.

12. An ostomy drainage system (1) according to any one of claims 2 to 11 wherein the mucus guard member (110) is between 0.8 and 1.2 cm long and preferably around 1cm long.

13. An ostomy drainage system (1) according to any one of the preceding claims wherein the connector body (104) is between 0.9 and 1.3 cm long and preferably around 1.1cm long.

14. An ostomy drainage system according to any one of claims 2 to 13, wherein a ratio of the length of the connector body (104) to the length of the mucus guard member (110) is between 1:1 and 1.5:1 and preferably around 1.1:1.

15. An ostomy drainage system (1) according to any one of the preceding claims wherein the connector device (102) provides an anchor that holds the connector device (102) connected to the valve (30) and a mucus guard member (110) that prevents / reduces how much mucus travels into the passage (106).

## Patentansprüche

1. Stomaentleerungssystem (1) mit einer Stomavorrichtung (10) und einer Entleerungsleitung (100), wobei die Stomavorrichtung (10) Folgendes aufweist:
ein inneres Sammelvolumen (20), das von einer ersten Wand (12) und einer zweiten Wand (14) definiert wird, die um einen Umfang miteinander verbunden sind;
eine Stomaaufnahmeöffnung (16), die in der ersten Wand (12) definiert ist, und
ein Ventil (30), das Folgendes aufweist:
einen mit der ersten und der zweiten Wand (12, 14) verbundenen Körper (32),
einen Einlass (40) und einen Auslass (42), die durch einen Durchflussweg verbunden sind und in Fluidverbindung mit dem inneren Sammelvolumen (20) stehen; und
wobei die Entleerungsleitung (100) eine Verbindervorrichtung (102) aufweist, die mit dem Ventil (30) verbindbar ist, wobei die Verbindervorrichtung (102) einen Verbinderkörper (104), der einen Durchgang (106) bereitstellt, und ein Einlassgebilde (111) hat, das sich relativ zu dem Verbinderkörper (104) radial nach außen aufweitet, so dass, wenn die Verbindervorrichtung (102) mit dem Ventil (30) verbunden ist, die Verbindervorrichtung (102) in den Auslass (42) des Ventils (30) eingeführt ist und der Verbinderkörper (104) sich vom Auslass (42) zum Einlass (40) erstreckt und sich durch den Einlass (40) in das Innere der Stomavorrichtung (10) erstreckt und das Einlassgebilde (111) sich nicht von dem Ventil (30) entfernen lässt.

2. Stomaentleerungssystem (1) nach Anspruch 1, wobei das Einlassgebilde (111) Teil eines Schleimschutzelements (110) bildet und, wahlweise, wobei, wenn die Verbindervorrichtung (102) mit dem Ventil (30) verbunden ist, der Verbinderkörper (104) sich vom Auslass (42) zum Einlass (40) erstreckt und das Schleimschutzelement (110) sich am Einlass (40) befindet und einen Käfig um den Einlass (40) bereitstellt, um das Eindringen von Schleim zu verhindern.

3. Stomaentleerungssystem (1) nach Anspruch 2, wobei das Schleimschutzelement (110) eine allgemein hochstehende Wand aufweist, die im Gebrauch vom Einlass (40) des Ventils (30) weg vorsteht, und, wahlweise, wobei die Wand in einer ringförmigen Form vom Verbinderkörper (104) vorsteht.

4. Stomaentleerungssystem (1) nach einem der Ansprüche 2 bis 3, wobei das Schleimschutzelement (110) eine Mehrzahl von Schlitzen oder Kanälen aufweist und, wahlweise, die Schlitze/Kanäle mit Abstand zueinander um den Einlass (40) herum angeordnet sind.

5. Stomaentleerungssystem (1) nach Anspruch 4, wobei das Schleimschutzelement (110) vier Schlitze hat und, wahlweise, wobei der erste und der zweite Schlitz durch einen Stab des Käfigs getrennt sind und ein erstes Paar bilden und der dritte und der vierte Schlitz durch einen zweiten Stab des Käfigs getrennt sind und ein zweites Paar bilden.

6. Stomaentleerungssystem (1) nach Anspruch 5, wobei das erste Paar sich auf einer dem zweiten Paar entgegengesetzten Seite des Schleimschutzelements (110) befindet.

7. Stomaentleerungssystem (1) nach einem der Ansprüche 2 bis 6, wobei das Schleimschutzelement (110) eine kegelstumpfförmige Form bildet, die an einer Basis breiter ist und sich zu einem Ende hin verjüngt, und, wahlweise, wobei die Basis zwischen 0,8 und 1,2 cm breit ist und vorzugsweise ungefähr 0,95 cm breit ist.

8. Stomaentleerungssystem (1) nach Anspruch 7, wobei das Ende des Schleimschutzelements (110) zwischen 0,7 und 0,95 cm breit ist.

9. Stomaentleerungssystem (1) nach einem der vorhergehenden Ansprüche, wobei der Verbinderkörper (104) ein Schultergebilde (111) aufweist, das im Gebrauch an den Auslass (42) des Ventils (30) angrenzend positioniert ist, und/oder wobei der Verbinderkörper (104) aus einem steifen Material und wahlweise aus einem steifen Plastikmaterial hergestellt ist.

10. Stomaentleerungssystem (1) nach einem der Ansprüche 2 bis 8 oder Anspruch 9, wenn ferner von einem der Ansprüche 2 bis 8 abhängig, wobei das Schleimschutzelement (110) aus einem steifen Material und wahlweise aus einem steifen Plastikmaterial hergestellt ist.

11. Stomaentleerungssystem nach Anspruch 10, wobei der Verbinderkörper (104) und das Schleimschutzelement (110) aus dem gleichen Material hergestellt sind.

12. Stomaentleerungssystem (1) nach einem der Ansprüche 2 bis 11, wobei das Schleimschutzelement (110) zwischen 0,8 und 1,2 cm lang ist und vorzugsweise ungefähr 1 cm lang ist.

13. Stomaentleerungssystem (1) nach einem der vorhergehenden Ansprüche, wobei der Verbinderkörper (104) zwischen 0,9 und 1,3 cm lang ist und vorzugsweise ungefähr 1,1 cm lang ist.

14. Stomaentleerungssystem nach einem der Ansprüche 2 bis 13, wobei ein Verhältnis der Länge des Verbinderkörpers (104) zur Länge des Schleimschutzelements (110) zwischen 1:1 und 1,5:1 ist und vorzugsweise ungefähr 1,1:1 ist.

15. Stomaentleerungssystem (1) nach einem der vorhergehenden Ansprüche, wobei die Verbindervorrichtung (102) einen Anker, der die Verbindervorrichtung (102) mit dem Ventil (30) verbunden hält, und ein Schleimschutzelement (110), das verhindert, dass Schleim in den Durchgang (106) gelangt, bzw. verringert, wie viel Schleim in den Durchgang (106) gelangt, bereitstellt.

## Revendications

1. Un système de drainage de stomie (1) comportant un appareil de stomie (10) et un conduit de drainage (100), l'appareil de stomie (10) comprenant :
un volume de collecte intérieur (20) défini par une première paroi (12) et une deuxième paroi (14) reliées l'une à l'autre sur un pourtour ;
une ouverture de réception de stomie (16) définie dans la première paroi (12), et
une valve (30) comprenant :
un corps (32) relié aux première et deuxième parois (12, 14), une entrée (40) et une sortie (42), reliées par un trajet d'écoulement et reliées fluidiquement au volume de collecte intérieur (20) ; et
le conduit de drainage (100) comportant un dispositif de raccord (102) qui peut être raccordé à la valve (30), le dispositif de raccord (102) ayant un corps de raccord (104) fournissant un passage (106), et une formation d'entrée (111) qui s'élargit radialement vers l'extérieur par rapport au corps de raccord (104), de manière à ce que, quand le dispositif de raccord (102) est raccordé à la valve (30), le dispositif de raccord (102) est inséré dans la sortie (42) de la valve (30), et le corps de raccord (104) s'étend de la sortie (42) à l'entrée (40) et rentre par l'entrée (40) dans l'intérieur de l'appareil de stomie (10), et la formation d'entrée (111) résiste au retrait de la valve (30).

2. Un système de drainage de stomie (1) selon la revendication 1, dans lequel la formation d'entrée (111) fait partie d'un élément de protection anti-mucus (110), et éventuellement dans lequel, quand le dispositif de raccord (102) est raccordé à la valve (30), le corps de raccord (104) s'étend de la sortie (42) à l'entrée (40) et l'élément de protection anti-mucus (110) est situé au niveau de l'entrée (40) et fournit une cage autour de l'entrée (40) pour empêcher l'entrée de mucus.

3. Un système de drainage de stomie (1) selon la revendication 2, dans lequel l'élément de protection anti-mucus (110) comprend une paroi généralement verticale qui, en cours d'utilisation, fait saillie de l'entrée (40) de la valve (30), la paroi faisant éventuellement saillie du corps de raccord (104) sous une forme annulaire.

4. Un système de drainage de stomie (1) selon une quelconque des revendications 2 à 3, dans lequel l'élément de protection anti-mucus (110) comprend une pluralité de fentes ou canaux, les fentes/canaux étant éventuellement espacés autour de l'entrée (40).

5. Un système de drainage de stomie (1) selon la revendication 4, dans lequel l'élément de protection anti-mucus (110) a quatre fentes, les première et deuxième fentes étant éventuellement séparées par un barreau de la cage et formant une première paire et les troisième et quatrième fentes étant séparées par un deuxième barreau et formant une deuxième paire.

6. Un système de drainage de stomie (1) selon la revendication 5, dans lequel la première paire est sur un côté opposé de l'élément de protection anti-mucus (110) par rapport à la deuxième paire.

7. Un système de drainage de stomie (1) selon une quelconque des revendications 2 à 6, dans lequel l'élément de protection anti-mucus (110) présente une forme tronconique qui est plus large à la base et s'amincit jusqu'à une extrémité, la base mesurant éventuellement entre 0,8 et 1,2 cm de large et de préférence environ 0,95 cm de large

8. Un système de drainage de stomie (1) selon la revendication 7, dans lequel l'extrémité de l'élément de protection anti-mucus (110) mesure entre 0,7 et 0,95 cm de large.

9. Un système de drainage de stomie (1) selon une quelconque des revendications précédentes, dans lequel le corps de raccord (104) comprend une formation en épaulement (111) en position adjacente à la sortie (42) de la valve (30) en cours d'utilisation, et / ou dans lequel le corps de raccord (104) est formé d'un matériau rigide et éventuellement un matériau de plastique rigide.

10. Un système de drainage de stomie (1) selon une quelconque des revendications 2 à 8, ou la revendication 9 quand elle dépend en sus d'une quelconque des revendications 2 à 8, dans lequel l'élément de protection anti-mucus (110) est formé d'un matériau rigide et éventuellement un matériau de plastique rigide.

11. Un système de drainage de stomie (1) selon la revendication 10, dans lequel le corps de raccord (104) et l'élément de protection anti-mucus (110) sont formés du même matériau.

12. Un système de drainage de stomie (1) selon une quelconque des revendications 2 à 11, dans lequel l'élément de protection anti-mucus (110) mesure entre 0,8 et 1,2 cm de long et de préférence environ 1cm de long.

13. Un système de drainage de stomie (1) selon une quelconque des revendications précédentes, dans lequel le corps de raccord (104) mesure entre 0,9 et 1,3 cm de long et de préférence environ 1,1cm de long.

14. Un système de drainage de stomie (1) selon une quelconque des revendications 2 à 13, dans lequel le rapport entre la longueur du corps de raccord (104) et la longueur de l'élément de protection anti-mucus (110) est entre 1:1 et 1,5:1 et de préférence environ 1,1:1.

15. Un système de drainage de stomie (1) selon une quelconque des revendications précédentes, dans lequel le dispositif de raccord (102) fournit un ancrage qui maintient le dispositif de raccord (102) raccordé à la valve (30) et un élément de protection anti-mucus (110) qui empêche/réduit la quantité de mucus qui circule dans le passage (106).
